(19) Europäisches Patentamt
European Patent Office
Office européen des brevets

(11) Publication number : 0 540 279 A1

# (12) EUROPEAN PATENT APPLICATION

(21) Application number : 92309799.2

(51) Int. Cl.$^5$ : C07H 13/06

(22) Date of filing : 26.10.92

(30) Priority : 29.10.91 GB 9122910

(43) Date of publication of application :
05.05.93 Bulletin 93/18

(84) Designated Contracting States :
CH DE ES FR GB IT LI NL SE

(71) Applicant : UNILEVER PLC
Unilever House Blackfriars P.O. Box 68
London EC4P 4BQ (GB)
(84) GB

(71) Applicant : UNILEVER N.V.
Weena 455
NL-3013 AL Rotterdam (NL)
(84) CH DE ES FR IT LI NL SE

(72) Inventor : Thornthwaite, David William,
Unilever Research
Port Sunlight Laboratory, Quarry Road East
Bebington, Wirral, Merseyside L63 3JW (GB)

(74) Representative : Bryant, Tracey et al
Unilever PLC Patent Division Colworth House
Sharnbrook
GB-Bedford MK44 1LQ (GB)

## (54) Process for preparing sugar esters.

(57) Process for preparing 1-acyl substituted acetylated sugars from fully acetylated sugars in a one step reaction.

EP 0 540 279 A1

This invention relates to a process for the manufacture of mixed esters of sugars, especially of pentoses and hexoses, usable as peroxyacid bleach precursors. In particular, the invention relates to the preparation of 1-acyl-2,3,4,6-tetraacetyl glucose, 1-acyl-2,3,4-triacetylxylose; 1-acyl-2,3,6,2[1],3[1],4[1],6[1]-heptaacetyl lactose; and 2-acyl-1,3,4,5-tetracetyl fructose.

Sugar derivatives consisting of a pentose or hexose sugar moiety, substituted with a long-chain acyl group and one or more short-chain acyl groups through ester bonds for use as peroxyacid bleach precursors, are described in WO 91/10719. Among these esters there is disclosed 2,3,4,6-tetra-O-acetyl-1-O-(long-chain acyl)-glucose [= 1-(long-chain acyl)-2,3,4,6-tetraacetyl glucose].

According to this reference, these esters are synthesized by methods known in the art, e.g. as described in WO 89/01480; D.Plusquellec **et al.**, Tetrahedron, Vol. 42, pp. 2457-2467, 1986; D.Plusquellec, Tetrahedron letters, Vol. 28, N° 33, pp. 3809-3812, 1987; J.M.Williams **et al.**, Tetrahedron 1967, Vol. 23. pp. 1369-1378; W.J.Hennen **et al.**, J. Org. Chem., Vol. 53, pp. 4939-4945 (1988); A.H.Haines, Adv. Carbohydrate Chem., Vol. 33, pp. 11-51, 1976; K.Joshimoto **et al.**, Chem. Pharm. Bull., 27 (11), pp. 2661-2674 (1979); and Z.Gyorgydeàk **et al.**, Liebigs Ann. Chem. 1987, pp. 235-241. None of these art methods specifically esterify the anomeric (1) position; most have the 1-position as $-OCH_3$ and esterify other hydroxyls, usually the primary hydroxyl at the 6-position.

Where esterification at the anomeric (1) position is mentioned, this is either achieved enzymatically, by using complex "protection group" chemistry, or by using complex chemical reagents.

The enzymatic route uses selective hydrolysis of glucose pentaacetate and other sugar acetates. Specifically, when porcine pancreatic lipase is used, the yields are variable, i.e. about 70% (the concentration of the sugar was approximately 10 g/l, or 1% solution using 10% dimethyl formamide in phosphate buffer). The lipase enzyme concentration was 0.75 g/mmol sugar and the mixture was stirred at 25°C (reaction monitored by TLC). The mixture was then extracted with ethyl acetate and purified by chromatography. Obviously, if this procedure is used for industrial manufacture, much work will be required. The main disadvantages of this process include the need for (i) dilution; (ii) the use of solvents such as dimethyl formamide; and (iii) chromatographic purification. The reaction time may also be a disadvantage.

The use of "protection group" chemistry works very well for laboratory synthesis but is very inefficient and expensive. This method involves benzyl protection of the anomeric position (yield approx 60%). The other hydroxy groups can then be acetylated to give, e.g., 1-benzyl-2,3,4,6 tetraacetyl glucose. The protecting group is then removed by hydrogenation (yield ~95%) and the resulting tetraacetate is acylated. This method involves at least 3 steps if the starting product is glucose and has a low yield for the benzylation step (60%). Furthermore, the 1-benzyl material requires purification.

Another art route involves the use of acyl transfer agents which selectively acylate the anomeric alcohol. This procedure has been used by Plusquellec **et al.** and. can produce 1-octanoyl glucose.

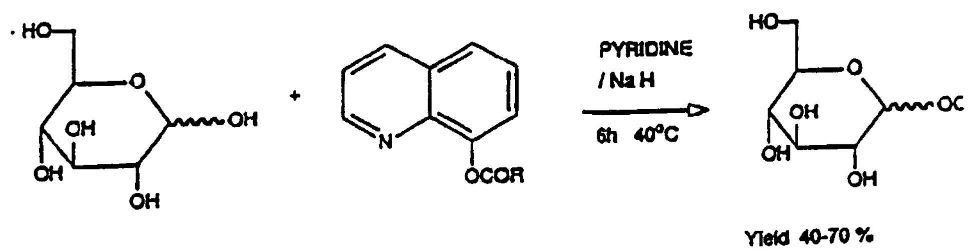


The reaction is ideal for laboratory synthesis but involves the expensive toxic solvent pyridine and sodium hydride as catalyst, known to be a very hazardous material. The reagent itself, 8-acyloxy quinoline, is said to be commercially difficult to obtain and expensive and yields based on acylating agent are 40-70%. (The sugar is used in 3 times excess, which is reported to be unattractive commercially).

Our copending British patent application No. 9116939.1 describes a process for preparing monoacyl substituted acetylated sugars from a fully acetylated sugar in a two-step process. The process involves removal of the anomeric 1-acetyl group followed by treatment of the resultant material with an acid chloride or anhydride. The disadvantage of this process is that it involves two steps.

Accordingly, it is an object of the present invention to provide an improved method for the preparation of 1-acyl-substituted acetylated sugars in high yields and with good conversion in one step.

A further object of the present invention is to provide a process for the preparation of 1-acyl-substituted acetylated sugars from commercially available and relatively inexpensive raw materials, with the advantage of being suitable for large-scale industrial operation.

Thus, accordingly the present invention provides a process for the preparation of 1-acyl-substituted acetylated sugars comprising reacting a fully acetylated sugar with a carboxylic acid in the presence of catalyst.

Preferred fully acetylated sugars for use in the present invention are 1,2,3,4,6-pentaacetyl glucose (PAG) and 1,2,3,4-tetraacetyl xylose (TAX), and 1,2,3,6,$2^1$,$3^1$,$4^1$,$6^1$ -octa acetyl lactose (OAL); and 1,2,3,4,5-pentaacetyl fructose.

Suitable catalysts for use in the present invention include metal salts; for example, Zn, Hg, Al, Fe, Co, Ni, Cu, Li, Cr, Mn, Cd, Sn, Ti and Zr salts, such as chlorides, acetates or bromides, i.e. Lewis acids comprising a transition metal, sodium bisulphate, sulphonic acid resins and toluene sulphonic acid.

Particularly preferred catalysts are zinc chloride, aluminium chloride, tin chloride and sodium bisulphate.

Any carboxylic acid other than acetic acid may be used in the present invention. Preferably, the carboxylic acid will contain at least four carbon atoms. It may be branched, unbranched, saturated, unsaturated, linear, aliphatic, alicyclic, aromatic or alkyl aromatic in nature. Particularly preferred carboxylic acids include benzoic and octanoic acid and those acids having either an ester or amide link.

Preferably, the reaction is carried out by heating a mixture of 1 mol-equivalent of the acetylated sugar, e.g. glucose pentaacetate, with 1-5 mol-equivalent of a carboxylic acid, e.g. benzoic acid or octanoic acid, to a temperature of from about 100°C to about 200°C, preferably about 120°C-150°C, and thereafter adding a catalyst (about 1 mol % to 50 mol %). Acetic acid, which is formed, is distilled off in vacuo at a temperature of about 100-150°C. The resulting reaction mixture is then washed with water to remove the catalyst.

This procedure gives a high yield in the range of 80% or greater by weight, and a good conversion in a one step process, taking about one hour at a reaction temperature of about 150°C. The material produced requires a small amount of purification and all ingredients are commercially available and relatively- inexpensive. A solvent is not needed in the preparation, although, it may be necessary to use one in any further purification step.

Mono-acyl substituted acetylated sugars prepared according to this method find particular application as bleach precursors for use in detergent compositions comprising a source of hydrogen peroxide such as, for example alkali metal peroxides, alkali metal perborates and, in particular, sodium perborate tetrahydrate and sodium perborate monohydrate; a surface active material; detergency builders and other common detergent composition additives and as described in our pending British Patent Application No. 9116939.1, incorporated herein by reference.

The following examples will more fully illustrate the embodiment of this invention without limiting the invention thereto.

## EXAMPLES

### Example I

Preparation 1-octanoyl-2,3,4-6-tetraacetyl glucose (OTAG)

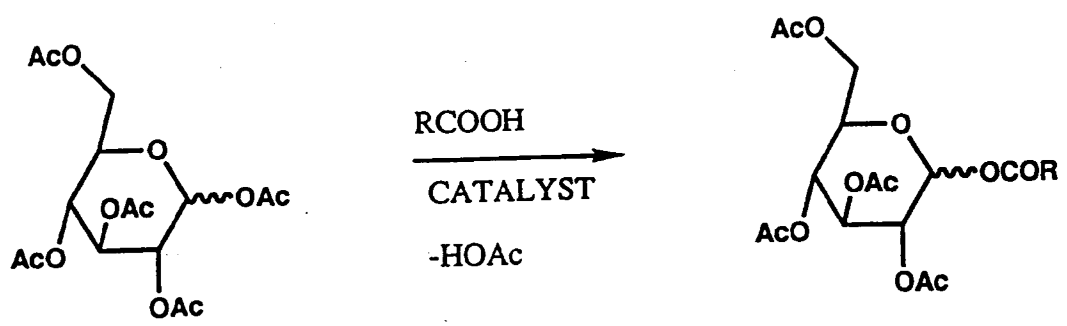


GPA

Pentaacetyl glucose (GPA) - (9.75 g; 0.025 mol) was mixed with octanoic acid (4.3 g; 0.03 mol), i.e. about 20% excess, and this mixture was heated to 120°C until a homogeneous liquid was obtained. Zinc chloride (0.5 g; 0.36 mMol = about 10 mol%; 5 wt.%) was added and the mixture was further heated under reduced pressure (6 mm Hg) and kept at 130°C for 15 minutes with subsequent distillation of the acetic acid formed. The reaction was followed by thin-layer chromatography silica/ether eluent.

OTAG Rf = 0.85

PAG Rf = 0.71

After distillation, the mixture was cooled and dissolved in ethyl acetate (100 ml) and washed with a sodium bicarbonate solution (10%; 100 ml), dried over sodium sulphate, filtered and the solvent was removed under reduced pressure to yield a sticky solid (11 g; 93%) : 'Hnmr (δ $CDCl_3$) .6.35 1H CH CO $C_7H_{15}$ (α-anomer) OTAG, 5.75 1H CH CO $C_7H_{15}$
[β-anomer] - OTAG; some PAG was also present ca. 7%.

Example II

Preparation of 1-octanoyl sugars using various catalysts (see Table 1) 1-octanoyl-2,3,4,6-tetraacetyl glucose

Glucose pentaacetate (10 g; 0.025 mol) was added to a round bottom flask (100 ml) equipped for vacuum distillation. Octanoic acid (4.3 g; 0.029 mol) was added. The mixture was then heated to 120°C to form a molten liquid and then the catalyst (0.5g) was added. The apparatus was placed under vacuo (1-10 millibar) and the temperature raised slowly to within the range 150-180°C. The acetic acid formed was removed by distillation. This reaction was carried out over a period of 15-40 mins depending of the heating rate and speed of acetic acid distillation. After removal of acetic acid the mixture was analysed by thin layer chromatography (silica gel Kieselgel 60 $F_{254}$(ex Merck)/Diethyl ether solvent) and the results obtained summarised in Table I. After distillation, the mixture was cooled and dissolved in ethyl acetate (100 ml) and decolourising charcoal (1-2 g) added. This mixture was refluxed for 10 mins filtered, cooled and washed with water (100 ml), saturated sodium chloride solution (50 ml) and dried over sodium sulphate. The dried solution was filtered and concentrated to dryness under vacuo to yield a 'sticky' solid (yields see Table I).

The results show a wide range of catalysts are useful in the reaction. It is particularly surprising that the acid catalyst sodium bisulphite works so well as it would be expected that this catalyst would promote transesterification on the 2,3,4 and more particularly the 6 position.

Table 1

| Catalyst | Max. Temp°C | % yield OTAG | % GPA | TLC (number of spots) | Comments on TLC/ Analysis[a)] OTAG | GPA | Others |
|---|---|---|---|---|---|---|---|
| $ZnOAc_2$ | 130 | 77 | 18 | 2 | Yes | Yes | - |
| $ZnCl_2$ | 130 | 76 | 20 | 2 | " | " | - |
| $FeCl_3$ | 170 | 75 | 15 | 4 | " | " | Yes[b)] |
| ZnO | 120 | 74 | 18 | 2 | " | " | - |
| $AlCl_3$ | 160 | 83 | 10 | 2 | " | " | - |
| $Zr(SO_4)_2$ | 140 | 74 | 15 | 2 | " | " | - |
| $SnCl_2$ | 130 | 87 | 8 | 2 | " | " | - |
| Amberlyst (ex Rohme Haas) ion exchange resin ($SO_3H$ form) | 130 | 75 | 15 | 2 | " | " | - |
| Tolulene sulphonic acid | 130 | 80 | 10 | 2 | " | " | - |
| $NaHSO_4$ | 130 | 87 | 8 | 2 | " | " | - |

a) All products were identified by TLC and $^1$Hnmr

b) Small amounts of other products detected but these were minor components.

## Example III

Preparation of 1-octanoyl-2,3,6,$2^1$,$3^1$,$4^1$-$6^1$ heptaacetyl lactose

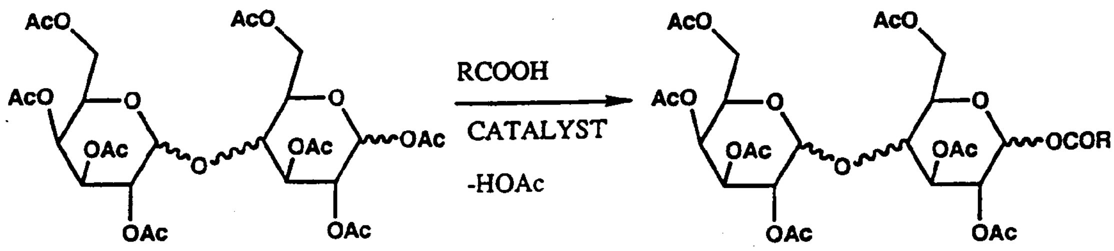


LACTOSE OCTAACETATE

α-D Lactose octaacetate was prepared by refluxing lactose in an excess of acetic anhydride with sodium acetate. A white solid was obtained which was recrystallised from ethanol mpt 150-151°C lit. mpt 152°C (Dictionary of Organic Compounds, Vol 4, page 1996, Eyre & Spottiswode Ltd London 1965). ν (nujol) 1750 $cm^{-1}$. TLC (Kieselgel 60 $F_{254}$; Diethyl ether) one spot Rf 0.21. $^1$Hnmr (δ; $CDCl_3$) 5.65 (d; 1H anomeric H - OAc); 5.35 (d, 1H CH-OAc); 5.25 (t, 1H, CH-OAc); 5.1 (m 2H 2 x CH C-O-); 4.95 (dd, 1H, CH-OAc); 4.5 (m, 2H, 2 x CH-O-); 4.1 (m, 3H, $CH_2$OAc, t CH-O) 3.85 (m, 2H, $CH_2$-OAc); 3.75 (m, 1H CH-OAc); 2.17, 2,13, 2.1, 2.07 2.05, 2.04, 2.03, 1.97 (all singlets 3H 8 x $COCH_3$).

Lactose octaacetate (3 g; 0.0045 mol) was added to octanoic acid (1.9 g, 0.013 mol) in a flask (50 ml) equipped for distillation. The mixture was heated to 130°C to form a molten mixture. Zinc chloride (0.2 g) was added and the resulting mixture further heated under reduced pressure (1 millibar) and kept at 130°C for 15 minutes while the acetic acid formed was distilled off.

The mixture was then cooled, dissolved in ethyl acetate (50 ml) and treated with decolourising charcoal filtered, washed with water (50 ml), sodium bicarbonate solution (50 ml), brine (50 ml) and dried over sodium sulphate. The mixture was then filtered and the solvent removed under reduced pressure to yield a solid (2.05 g; 50% yield). TLC (Diethyl ether) (1 spot Rf 0.31). $^1$Hnmr (δ; $CDCl_3$) 6.26 (d, 1H β anomer) 5.65 (d, 1H α anomer); 2.45 (t, 2H R-$CH_2$ CO β anomer), 2.35 (t, 2H R-$CH_2$ CO α anomer) 2.17, 2.13, 2.08, 2.07, 2.03, 2.00, 1.97 (3H, S, 7x $COCH_3$), 1.68 (t, 2H, $CH_2$ $CH_2$-CO), 1.3 (m, 8H, $(CH_2)_4$-$CH_2CH_2$CO), 0.9 (t, 3H, $CH_3$-$CH_2$).

## Example IV

Preparation of 2-octanoyl-1,3,4,5 tetraacetyl fructose

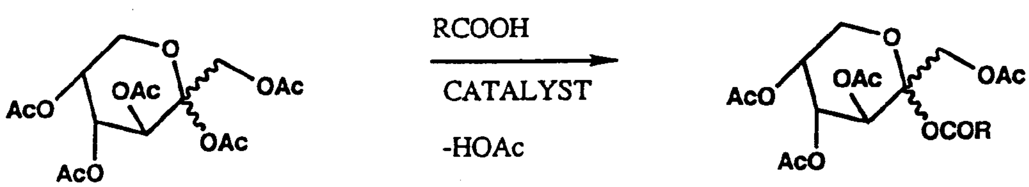


FRUCTOSE PENTAACETATE

Fructose was converted to its penta acetate using acetic anhydride and sodium acetate to yield a viscous oily solid (ν nujol) 1745 $cm^{-1}$ TLC (diethyl ether/Kieselgel 60 $F_{254}$) one spot Rf 0.65.

Fructose pentaacetate (3.9 g; 0.01 mol) was added to octanoic acid (1.9 g; 0.013 mol) and heated to 120° to form a molten mixture in a flask (50 ml) equipped for vacuum distillation. Zinc chloride (0.2 g) was added and the acetic acid which formed removed under reduced pressure (1 millibar). The mixture was cooled, dissolved in ethyl acetate (50 ml), boiled with activated charcoal, filtered washed with water (50 ml), sodium bicarbonate solution (50 ml) and brine (50 ml). The ethyl acetate was then dried over sodium sulphate, filtered and concentrated under reduced pressure to yield a 'sticky' solid (3.4 g; 72%) TLC (Diethyl ether) 2 spots Rf 0.73, 0.65 very weak (fructose pentaacetate).

Example V

Preparation of 1-acyl -2,3,4,6 tetraacetyl glucose

Glucose pentaacetate (5 g; 0.0125 mol) was added to a flask (50 ml) equipped for vacuum distillation and to this flask was added various carboxylic acids (see Table II) (0.015 mol). The mixture was heated to 130°C to form a melt. Zinc chloride (0.2 g) was added and the mixture further heated under reduced pressure (1-10 millibar) with consequent removal of the acetic acid. The resulting mixtures were cooled, dissolved in ethyl acetate (50 ml) treated with decolouring charcoal, filtered, washed with water (50 ml), sodium bicarbonate solution (50 ml) and brine (50 ml). The ethyl acetate was dried over sodium sulphate, filtered and concentrated under reduced pressure to yield solid products. The results obtained are summarised in Table II.

Preparation of N-octanoyl glycine

Glycine (11.25 g; 0.15 mol) was dissolved in water (50 ml), sodium hydroxide (12 g 0.3 mol) added and the mixture stirred. To this solution octanoyl chloride (24.4 g; 0.15 mol) in acetone (50 ml) was added dropwise with vigourous stirring over 1 hour. The mixture was further stirred for 1 hour at room temperature and, thereafter, concentrated to dryness under reduced pressure to give a white solid. This solid was boiled with ether (3 x 150 ml); filtered and then the combined ethereal layers washed with brine (150 ml). It was then dried over sodium sulphate filtered and concentrated under vacuo. The resulting white solid (27 g, 83% yield) mpt 105-106°C was used in the experiment described in Table II. $^1$Hnmr ($\delta$, $CD_3OD$) 3.9 (S, 2H, HN $\underline{CH_2}CO_2H$), 2.25 (t, 2H, $\underline{CH_2}$ CONH), 1.6 (t, 2H, $\underline{CH_2}$-$CH_2$ CO, 1.3 (m, 8H,-$\underline{(CH_2)_4}$-$CH_2CH_2CO$), 0.9(t, 3H, $\underline{CH_3}$-$(CH_2)_4$).

Preparation of 2-Octanoyl glyoxylic acid

Glyoxylic acid (11.4 g 0.15 mol) was added to acetonitrite (150 ml) containing triethyl amine (30.3 g; 0.3 mol) and to this solution was added octanoyl chloride (24.4 g; 0.15 mol) dropwise with stirring and cooling (temp 25°C) over a period of 1h. The resulting mixture was refluxed for 1 hour and the solvent was removed under vacuo to yield a white solid. This solid was partitioned between hydrochloric acid (150 ml; 5M) and ether (300 ml). The ethereal layer was washed with water (100 ml), sodium bicarbonate (2 x 50 ml) and dried over sodium sulphate.

The sulphate was removed by filtration and the ether concentrated under vacuo to yield an oil which was distilled under reduced pressure 180-184°C (10 millibar) giving a white solid which was recrystallised from petroleum ether 60/80 boiling range) (6.3 g; 21% yield) glc 98% $^1$Hnmr ($\delta_3$, $CDCl_3$) 4.2 (s, 2H, $\underline{CH_2}CO_2H$), 2.43 (t, 2H, -$\underline{CH_2}$ -COO) 1.6 (t, 2H $\underline{CH_2}$-$CH_2COO$), 1.3 (m, 8H, $CH_3\underline{(CH_2)_4}$-$CH_2$), 0.9 (t, 3H, $\underline{CH_3}$-$(CH_2)_4$).

Table II

Preparation of 1-Acyl-2,3,4,6-tetraacetyl Glucose Using Various Carboxylic Acids and Zinc Chloride Catalyst

| Carboxylic acid | Max. temp. °C | % Yield Product | TLC | Comments[a*] |
|---|---|---|---|---|
| Benzoic acid | 130 | 82 | one spot | product only |
| N-octanoyl glycine $C_7H_{15}CONHCH_2CO_2H$[(b)] | 130 | 76 | two spots | product major GPA (minor) |
| 2-octanoyl glyoxylic acid, $C_7H_{15}COOCH_2CO_2H$[(c)] | 130 | 76 | two spots | product major GPA (minor) |

(b),(c) preparations described below.

a*) all products identified by TLC & $^1$Hnmr

## Claims

1. A process for preparing 1-acyl substituted acetylated sugars comprising reacting a fully acetylated sugar with a carboxylic acid other than acetic acid in the presence of a catalyst.

2. A process according to claim 1 wherein the carboxylic acid contains at least four carbon atoms.

3. A process according to claim 2 wherein the carboxylic acid is selected from acids having an ester link, an amide link, benzoic acid and octanoic acid.

4. A process according to claim 1 wherein the catalyst is selected from Lewis acids comprising a transition metal; sodium bisulphate; sulphonic acid resins; and toluene sulphonic acid.

5. A process according to claim 1 wherein the fully acetylated sugar is selected from 1,2,3,4,5-pentaacetyl fructose, 1,2,3,4,6-pentaacetyl glucose, 1,2,3,4-tetraacetyl xylose and 1,2,3,6,$2^1$,$3^1$,$4^1$,$6^1$-octaacetyl lactose.

European Patent Office

# EUROPEAN SEARCH REPORT

Application Number

EP 92309799.2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| D,A | WO - A - 91/10 719 (NOVO NORDISK A/S) * Abstract, page 3, lines 13-16; page 4, lines 1-14; claims 1,4,5,11,18-20 * -- | 1-3,5 | C 07 H 13/06 |
| D,A | THE JOURNAL OF ORGANIC CHEMISTRY, vol. 53, 1988, W.J. HAMEN et al. "Enzymes in Carbohydrate Synthesis: Lipase-Catalyzed Selective Acylation and Deacylation of Furanose and Pyranose Derivatives", pages 4934-4945 * Abstract; table III * -- | 1,5 | |
| A | C.R. NOLLER "Lehrbuch der Organischen Chemie", 1960, Springer-Verlag, Berlin, Göttingen, Heidelberg, pages 396-397 * Page 397 * ---- | 1,4,5 | TECHNICAL FIELDS SEARCHED (Int. Cl.5) C 07 H 13/00 |

The present search report has been drawn up for all claims

| Place of search | Date of completion of the search | Examiner |
|---|---|---|
| VIENNA | 17-12-1992 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)